# EUROPEAN PATENT SPECIFICATION

(11) **EP 4 172 252 B1**
(45) Date of publication and mention of the grant of the patent: **28.08.2024**
(21) Application number: 21734365.6
(22) Date of filing: 29.06.2021
(51) Int. Cl.: C08J 7/16, C09D 165/00, C08G 61/12, C07D 487/22

(54) **MULTIPLY FUSED PORPHYRIN POLYMER FILM COATED ON A SUBSTRATE**
MEHRFACH FUSIONIERTER PORPHYRINPOLYMERFILM MIT EINER BESCHICHTUNG AUF EINEM SUBSTRAT
FILM DE POLYMÈRE DE PORPHYRINE MULTI-CONDENSÉE REVÊTU SUR UN SUBSTRAT

(30) Priority: 29.06.2020 LU 101891
(43) Date of publication of application: 03.05.2023
(73) Proprietor: Luxembourg Institute of Science and Technology (LIST), 4362 Esch-sur-Alzette (LU)
(72) Inventor: BOSCHER, Nicolas, 4363 ESCH-SUR-ALZETTE (LU); BABA, Kamal, 4363 ESCH-SUR-ALZETTE (LU); BENGASI, Giuseppe, 4363 ESCH-SUR-ALZETTE (LU); HUERTA FLORES, Ali Margot, 4363 ESCH-SUR-ALZETTE (LU)
(74) Representative: Lecomte & Partners
(86) International application number: PCT/EP2021/067772
(87) International publication number: WO 2022/002891

(56) References cited:
- KAMAL BABA ET AL: "Conductive Directly Fused Poly(Porphyrin) Coatings by Oxidative Chemical Vapour Deposition - From Single- to Triple-Fused : Conductive Directly Fused Poly(Porphyrin) Coatings by Oxidative Chemical Vapour Deposition - From Single- to Triple-Fused", EUROPEAN JOURNAL OF ORGANIC CHEMISTRY, vol. 2019, no. 13, 9 April 2019 (2019-04-09), DE, pages 2368 - 2375, XP055609416, ISSN: 1434-193X, DOI: 10.1002/ejoc.201900045
- MICHELE TONEZZER ET AL: "Production of novel microporous porphyrin materials with superior sensing capabilities", JOURNAL OF MATERIALS CHEMISTRY, vol. 22, no. 12, 1 January 2012 (2012-01-01) - 2012, GB, pages 5647, XP055343738, ISSN: 0959-9428, DOI: 10.1039/c2jm15008e
- DATABASE WPI Week 201989, 2019 Derwent World Patents Index; AN 2019-948819, XP002802290
- GIUSEPPE BENGASI ET AL: "Conductive Fused Porphyrin Tapes on Sensitive Substrates by a Chemical Vapor Deposition Approach", ANGEWANDTE CHEMIE INTERNATIONAL EDITION, vol. 58, no. 7, 11 February 2019 (2019-02-11), pages 2103 - 2108, XP055609430, ISSN: 1433-7851, DOI: 10.1002/anie.201814034
- NICOLAS D. BOSCHER ET AL: "Chemical vapour deposition of metalloporphyrins: a simple route towards the preparation of gas separation membranes", JOURNAL OF MATERIALS CHEMISTRY A, vol. 4, no. 46, 1 January 2016 (2016-01-01) - 2016, GB, pages 18144 - 18152, XP055343714, ISSN: 2050-7488, DOI: 10.1039/C6TA08003K
- BABA KAMAL ET AL: "Fused Metalloporphyrin Thin Film with Tunable Porosity via Chemical Vapor Deposition", APPLIED MATERIALS & INTERFACES, vol. 12, no. 33, 19 August 2020 (2020-08-19), US, pages 37732 - 37740, XP055828136, ISSN: 1944-8244, [retrieved on 20210727], DOI: 10.1021/acsami.0c09630
- IM SUNG GAP ET AL: "Conformal Coverage of Poly(3,4-ethylenedioxythiophene) Films with Tunable Nanoporosity via Oxidative Chemical Vapor Deposition", ACS NANO, AMERICAN CHEMICAL SOCIETY, US, vol. 2, no. 9, 1 January 2008 (2008-01-01) - 2008, pages 1959 - 1967, XP002606397, ISSN: 1936-0851, [retrieved on 20080828], DOI: 10.1021/NN800380E

## Description

The invention relates to a multiply fused polymer porphyrin film coated on a substrate, and a process for preparing same.

Porphyrins, composed of four pyrrole subunits connected by methine bridges, present numerous fascinating properties, including a strong aromaticity responsible for attractive absorption and emission properties and a rich metal coordination chemistry that have made them the catalysts of choice in main processes allowing life, such as photosynthesis and respiration. Several studies have reported the cooperative effect promoted by conjugated covalent links between porphyrins. Particularly, conjugated porphyrin arrays or polymers own superior functional properties, such as an increased catalytic activity. Among the conjugated porphyrin systems, directly fused porphyrin polymer tapes exhibit even more fascinating properties with a NIR (near infrared) absorption, two-photon absorption and negative attenuation factors.

Up to recently, the synthesis of fused porphyrin polymer tapes involved the solution-based dehydrogenative coupling of porphyrins using adequate oxidants. Nevertheless, the very poor solubility of these compounds limits the potential substituents to bulky solubilizing groups and prevent their integration into functional devices. Several studies have also reported the formation of fused porphyrin polymer tapes from the sublimation under ultra-high vacuum onto oriented metal surfaces. However, in the latter approach is solely yielding the formation of sub-monolayers and is completely dependent on the underlying substrate, preventing the fabrication of functional devices.

Plasma enhanced chemical vapor deposition (PECVD) is a process whereby the CVD reaction is ensured by interactions with chemically active high-energy species of the plasma. The wide variety of possibly highly reactive plasma species, i.e. with very low selectivity, was shown responsible for the disruption of most of the porphyrin macrocycles, such as expressed by the broad and low intensity of the Soret band in the UV/Vis spectra of the films (K. Nakamura, M. Watanabe, M. Zhou, M. Fujishima, M. Tsuchiya, T. Handa, S. Ishii, H. Noguchi, K. Kashiwagi and Y. Yoshida, Thin Solid Films, 1999, 345, 99). The sensing properties of the formed materials, called plasma polymers, rely on few remaining unaltered and non-covalently bonded porphyrins trapped in the plasma polymer scaffold as revealed by electrospray ionization mass spectrometry (ESI-MS) (M. Tonezzer, G. Maggioni and E. Dalcanale, J. Mater. Chem., 2012, 22, 5647). Due to the wide variety of highly reactive plasma species, highly branched and randomly terminated porphyrin-based plasma polymers are formed. The non-selectivity of plasma is responsible for the poor retention of the porphyrin macrocycle and the wide variety of bonding arrangements. Consequently, no multiply fused, conjugated, porphyrin polymers are obtained according to such plasma technology.

In order to circumvent the poor solubility of fused porphyrin polymer tapes, the Applicant very recently developed a chemical vapour deposition (CVD) method towards the simultaneous synthesis, deposition and doping of fused porphyrin thin films. In this method, the porphyrin and the oxidant are both delivered through the vapour phase onto a substrate on which they adsorb and react to form mainly multiply-fused porphyrin polymer tapes. While solution-based approaches produce fused porphyrin oligomers in moderate yields even with the aid of bulky substituents to suppress undesired aggregation, the oxidative chemical vapour deposition (oCVD) reaction of porphyrins ensures excellent yields of polymerization with almost no porphyrin monomer left in the coatings. The effective polymerization is expressed by the electrical conductivity of the thin films that reaches up to 1 S·cm⁻¹ and a strong light absorption in the visible to near-infrared spectral region.

Nevertheless, the advantages related to the oCVD of porphyrins go far beyond its unique ability to form fused polymer porphyrin thin films. Indeed, processing through the gas phase allows to circumvent the limitation related to the poor solubility of fused porphyrins and to decouple the porphyrin substituents from the synthesis requirements. This enables the use of smaller substituents, i.e. phenyl, which ensures the formation of dense and homogeneous thin films with superior conductivity. In addition, the higher reactivity of the porphyrins in oCVD enables the synthesis of fused polymer porphyrins chelating different metal cations, i.e. Co(II), Ni(II), Cu(II), Zn(II) and Pd(II). The synthesis of porphyrin dimers, oligomers and porphyrin polymer tapes relies on the oxidative coupling of porphyrins with free *β* and meso positions. This can lead to the formation of doubly or triply linked porphyrins in solution-based syntheses. In solution-based oxidative C-C coupling reactions, the regioselectivity towards doubly or triply-linked porphyrin tapes depends on the porphyrin metal center, as disclosed in WO 2020/099385 and in Bengasi et al. (G. Bengasi, L. Quétu, K. Baba, A. Ost, J. P. Cosas Fernandes, P. Grysan, K. Heinze, and N. D. Boscher, "Constitution and Conductivity of Metalloporphyrin Tapes", Eur. J. Inorg. Chem. 2020, 1-9). Up to date, oligomeric directly fused porphyrins with Zn(II), Ni(II), Pd(II) or Cu(II) as central metal ions prepared via solution-based approaches have been reported.

KAMAL BABA ET AL, "Conductive Directly Fused Poly(Porphyrin) Coatings by Oxidative Chemical Vapour Deposition - From Single- to Triple-Fused : Conductive Directly Fused Poly(Porphyrin) Coatings by Oxidative Chemical Vapour Deposition - From Single- to Triple-Fused", EUROPEAN JOURNAL OF ORGANIC CHEMISTRY, DE, (20190409), vol. 2019, no. 13, doi:10.1002/ejoc.201900045, ISSN 1434-193X, pages 2368 - 2375, discloses a simultaneous synthesis and deposition of conductive directly fused poly(porphyrin) coatings based on a substrate independent and up-scalable oxidative chemical vapor deposition (oCVD) approach.

However, none of the cited prior art documents specifically focus on the need to provide a fused polymer porphyrin film, obtainable by oCVD, exhibiting enhanced at least one of catalytic effects, namely heterogeneous electrocatalytic, heterogeneous photoelectrocatalysis or heterogeneous photocatalytic properties, and high surface area.

The invention relates to a multiply fused, conjugated, porphyrin polymer film coated on a substrate, said multiply fused, conjugated, porphyrin polymer being of formula (I) 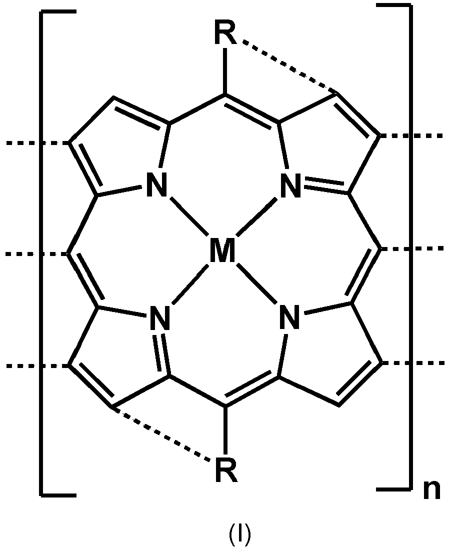 wherein
"n" is an integer greater than 1;
the porphyrin monomer units are di-meso-substituted porphyrins;
M is a metal cation selected from the group consisting of Mg(II), Fe(III), Co(II), Ni(II), Cu(II), Zn(II), Ru(II), Pd(II), Ag(II), Pt(II) and Au(III), or mixtures thereof;
the porphyrin units are multiply fused, including doubly-fused and/or triply-fused;
each substituent R attached to the meso position of the porphyrin monomer is an aromatic group presenting at least one free *ortho* position;
at least one of the two free *ortho* positions of the aromatic substituent R is fused to the *β* position of the porphyrin monomer,
wherein said porphyrin polymer film is a porous porphyrin polymer film with mean pore diameters, determined by SEM, within the range of from 2 nm to 100 nm and exhibiting a density, calculated as disclosed in the description, not greater than 2 g/cm³.

The Applicant took advantage of the oCVD to preliminary investigate the dehydrogenative coupling of porphyrins at different temperatures. Indeed, while the solution-based coupling of porphyrins is usually undertaken at room temperature and cannot be performed at significantly higher temperature due to the use of organic solvent, oCVD authorised the study of the dehydrogenative coupling of porphyrins at temperature as high as 250°C.

In the context of the invention, the expressions "porous fused, conjugated, porphyrin polymer films", "porous polyporphyrin(s)", "porous polyporphyrin film(s)", "polyporphyrin film(s)" and the like, have the same meaning.

In the film of the invention, the porphyrin polymer is composed of structures wherein one porphyrin monomer repeating unit is directly fused to another one porphyrin monomer repeating unit by at least one C-C linking. It could be easily deduced from the structure of the polyporphyrins that there are some conjugated structures.

A first aspect consisted to elucidate the influence of the substrate temperature on the chemistry and/or synthesis of the fused porphyrin polymer films, especially on the growth rate, morphology and nanomechanical properties thereof.

The reaction temperature, *i.e.* substrate temperature, drastically affects the chemistry and morphology of fused porphyrin polymer films prepared from the oCVD. Increase of the reaction temperature notably induces a higher degree of conjugation of the fused porphyrin chains, which may be evidenced by high resolution mass spectrometry. While the oCVD film elaborated at room temperature are mostly singly-fused porphyrins very generally, the one elaborated at 200°C is, very generally, mostly composed of multiply-fused porphyrins. Besides, higher substrate temperatures very often promote the chlorination of the fused porphyrins, known to decrease the solubility of porphyrins.

A second important aspect to provide such fused porphyrin polymer films is the nature and the quantity of oxidant used, vs porphyrin monomer starting reactant, in oCVD. It has been reported that the oxidant is generally used in excess to ensure sufficient coupling efficiency, for example of from 20 to 28 fold excess (Bengasi *et al.* previously cited), and FeCl₃ demonstrated to be a good choice to promote coupling of porphyrins over their undesired chlorination.

The Applicant has surprisingly found that a specific combination of both temperatures range and oxidant/starting porphyrin monomer reactant ratio range, allows to obtain such porous fused, conjugated, porphyrin polymer films. Photocatalytic and/or electrocatalytic effects, especially photocatalytic hydrogen or oxygen evolution from water splitting, may be of about 2-5 fold higher than those when invention conditions are not fulfilled. High porosity enables water to penetrate the porous structure of the porous polyporphyrin film(s) elaborated at higher temperatures in a greater extent, i.e.; higher water uptake. The polyporphyrin film(s) may advantageously be used in sensing and catalytic devices and/or materials.

According to the invention, porphyrin units are multiply fused, including doubly-fused, i.e *β-meso, meso-β,* and triply-fused, i.e *β*-*β*, meso-meso, *β-β.*

Each substituent R, which are preferably two substituents, are advantageously in positions 5 and 15.

Advantageously, M may be selected from Co(II), Ni(II) and Cu(II), or mixture thereof, as providing better photocatalytic and/or electrocatalytic effects.

Advantageously, the mean pore diameters may be within the range of from 5 nm to 80 nm, more preferably of from 10 nm to 50 nm and, independently, the density may be of from 0,20 g/cm³ to 1,5 g/cm³, more preferably of from 0,20 g/cm³ to 1,2 g/cm³, most preferably of from 0,25 g/cm³ to 1 g/cm³. Such densities provide the best catalytic properties of the porous polyporphyrin(s).

The film's density (g.cm⁻³) is calculated from the measured surface density and the thickness of each film. The surface density in some more precise aspects may be assessed from the specific mass gain due to the deposition and the area of the coated surface: Surface Density [µg·cm⁻²] = (m₁-m₀)/A, where m₀ and m₁ are the masses of the coated and uncoated substrate, respectively, while A is the coated area estimated using ImageJ software. Coated microscope glass slides is weighted using a microbalance to obtain m₁, then the samples were immersed in a mixture of acetone/ethanol (50:50 %) for 2 min before to be cleaned using Kimtech tissues to remove all the deposited film and obtain m₀. The surface density measurements were repeated on three different samples for each deposition condition to determine the average surface density.

The porous structure, and especially the diameters and *in fine* the mean pore diameters, of the fused porphyrin tapes thin film with numerous voids or pores is evidenced by SEM. In the context of the invention, even a mesoporous structure may be obtained. It is known in the art that a mesoporous material is a material containing pores having generally diameters of from to 2 and 50 nm, according to IUPAC nomenclature. General considerations are that pores come in a variety of sizes to address a very wide range of applications. When considering pore size, a set of standards approved by IUPAC has defined pore size ranges based on different size widths (Sing, S. W., et al, (1985). Reporting physisorption data for gas/solid systems with special reference to the determination of surface area and porosity, Pure Appl. Chem. 57, 603). Pores with an internal width of less than 2 nm are referred to as micropores, those with an internal width between 2 nm and 50 nm as mesopores, and those larger than 50 nm are called macropores.

The n value of the porphyrin repeating units in the polyporphyrins may preferably be of from 2 to 50, more preferably of from 10 to 50, most preferably, of from 10 to 40. Such n value ranges, in combination with the mean pore diameters and the density of the porous polyporphyrin, are the best for obtaining the desired technical effect.

Advantageously, the porous polyporphyrin(s) may also present at least one of the following characteristics:
- a thickness of from 500 nm to 10 µm, the higher thickness advantageously increasing the catalytic properties. More preferably, the thickness may be within a range of from 500 nm to 5 µm, especially of from 500 nm to 2 µm.
- an electrical conductivity of from 10⁻⁴ S/cm to 10 S/cm;
- a Young's modulus of from 1 GPa to 3 GPa; the decrease of the film's density is coupled to a decrease of the Young's modulus;
- a water uptake of from 0,5 mg/mm³ of H₂O to 1,0 mg/mm³ of H₂O, more preferably of from 0,8 mg/mm³ of H₂O to 1,0 mg/mm³ of H₂O.

Preferably, the aromatic group (R) may be a 6-membered aromatic ring structure selected from the group consisting of monovalent radicals of benzene and pyridine, more preferably being phenyl, 4-pyridyl and 3-pyridyl, or mixtures thereof.

In some embodiments, the aromatic group may have one or more substituents at *para* and *meta* positions thereof, and may be selected from the group consisting of nitro, amino, hydroxyl, sulfonyl, halogen atom, such as Cl, Br and F, alkyl, haloalkyl, hydroxyalkyl, aminoalkyl or alkoxy groups, wherein the alkyl is a in C₁-C₆ alkyl group, preferentially forming 4-methylphenyl (p-tolyl), 3,5-di-tert-butylphenyl, 4-hydroxyphenyl, 4-carboxyphenyl, 4-aminophenyl, 3-cyanophenyl, 4-trifluoromethyl-phenyl, 4-fluorophenyl, 3,5-bis(trifluoromethyl)phenyl, 4-chlorophenyl and 4-bromophenyl groups, or mixtures thereof.

In some instances, when the aromatic group is a 6-membered aromatic ring, it may be fused at *para* and *meta* positions thereof to other 6-membered aromatic ring, preferentially forming naphthalene, anthracene, phenanthrene or pyrene groups, or mixtures thereof.

The substrate is preferentially an electrically conductive substrate or presents at least a part of an electrically conductive surface, preferentially fluorine-doped tin oxide (FTO) or indium-doped tin oxide (ITO) coated glass or polyethylene terephthalate (PET) substrates, titanium, copper, platinum, stainless steel, gallium arsenide (GaAs), gallium phosphide (GaP), silicon carbide (SiC), sapphire, stainless steel.

The invention also concerns a process for forming, on a substrate, a multiply fused, conjugated, porphyrin polymer of formula (I) according to the invention, the method comprising the steps of:
(a) providing a substrate in a vacuum chamber,
(b) performing on said substrate an oxidative chemical vapour deposition reaction with a predetermined quantity of at least one oxidant selected from FeCl₃, CuCl₂ and Cu(ClO₄)₂, and a predetermined quantity of at least one porphyrin monomer of general formula (II): 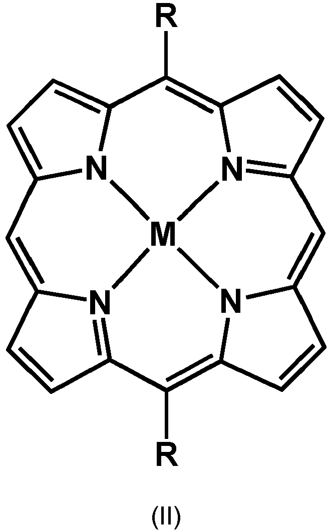 wherein
   - M is a metal cation selected from the group consisting of Mg(II), Fe(III), Co(II), Ni(II), Cu(II), Zn(II), Ru(II), Pd(II), Ag(II), Pt(II) and Au(III), or mixtures thereof;
   - each substituent R attached to the *meso* position of the porphyrin monomer is an aromatic group presenting at least one free *ortho* position;
   wherein step (b) is carried at a substrate temperature range of the substate of from 150°C to 250°C, and in that the ratio (*r*) of the at least one oxidant molar quantity/at least one porphyrin monomer molar quantity is within a range of from 35 to 150.

As above mentioned, the Applicant first investigated the reaction temperature, *i.e.* substrate temperature, and shown that the temperature drastically affects the chemistry and morphology of fused porphyrin polymer films prepared from the oCVD. Increase of the reaction temperature (substrate temperature) notably induces a higher degree of conjugation of the fused porphyrin chains, that may be evidenced by high resolution mass spectrometry. While the oCVD thin film elaborated at room temperature are mostly singly-fused porphyrins, the one elaborated at 200°C are mostly composed of doubly-fused porphyrins. Besides, higher substrate temperatures also promote the chlorination of the fused porphyrins, known to decrease the solubility of porphyrins.

Moreover, by performing the oCVD, the increase of the substrate temperature drastically decreases the density of the polyporphyrin(s) films to be not higher than 2 g/cm³. For instance, setting the substrate temperature at 25°C, the density of a given polyporphyrin film may be of about 4,5 g/cm³, at 200°C, the density of the same film may be of about 0,25 g/cm³.

The increase of the reaction temperature (substrate temperature) within the range of temperature of from 50°C to about 100°C of the prior art, results generally in a linear increase of the polyporphyrin film thickness, for example of from about 100 nm to 300 nm for a 30 minutes deposition under selected deposition conditions. The Applicant has shown that a geometric growth of the thickness is observed within the temperature range of about 150°C to 250°C, for example of from about 550 nm to 1700 nm for 30 minutes deposition under the same deposition conditions.

A second important aspect to provide such fused porphyrin polymer films is the nature and the quantity of oxidant used, vs porphyrin monomer starting reactant, in oCVD, as explained above.

The Applicant has surprisingly found that a synergic effect is observed with a specific combination of both the reaction temperature (substrate temperature) range of from 150°C to 250°C and oxidant molar quantity/starting porphyrin monomer molar quantity ratio r within a range of from 35 to 150, and said combination is effective to attain such porous fused porphyrin polymer films having mean pore diameters of from 2 nm to 100 nm, and a density as low as 2 g/cm³.

Without being bound with any theory, it may be assumed that a large excess of said oxidant quantity generates undesirable oxidant cluster agglomerates in the layer of polyporphyrins. Said agglomerates have an impact on polyporphyrin mechanical and electrical properties explained by the fact that said clusters agglomerates are not covalently linked to the polyporphyrins, thereby they may be dissolved in water or in another polar solvent, especially organic polar solvent, and are not inducing conductivity. Moreover, the oxidant excess induces an undesirable excessive chlorination of porphyrins and polyporphyrin chains which affects or comes in competition with the polymerization reaction. Indeed, when the Cl atom takes place of an H in the porphyrin monomer, the dehydrogenative coupling (or polymerization and intramolecular cyclisation), cannot be performed at the Cl substituted position.

The synergic effect is observed because even if an excess of the oxidant reaches the surface of the polyporphyrin film, the high temperature range (150°C-250°C):
(i) partly desorbs the oxidant which accumulates at the surface, and with release of a greater amount of gaseous FeCl₃ by-products, e.g. Cl₂, while imparting
(ii) an enhanced reactivity for the dehydrogenative coupling, releasing a greater amount of HCI.

For *r* > 150, a collapse of the photocatalytic activity is observed. Such a decrease of the photocatalytic properties may be attributed to the cracking and decohesion of the polyporphyrin film(s) related to the excess of oxidant and oxidant by-products trapped into the thin films and dissolved upon immersion in aqueous media.

Although the chlorination is maximal at temperatures of about 100°C, surprisingly said chlorination decreases at 200°C, whereas polymerization and intramolecular cyclisation still increases.

More preferably, the *r* range is of from 45 to 125, most preferably of from 50 to 100. Said ranges, independently and in combination with the temperature ranges as set before, are those providing a low density polyporphyrin films of from 0,25 g/cm³ to 1 g/cm³ concomitantly with the best catalytic effects. As an example, the hydrogen photocatalytic production, from water splitting, using porous polyporphyrin(s) films may be higher than about 5 µmol.cm⁻².h⁻¹, even higher than about 9 µmol.cm⁻².h⁻¹, especially higher than 20 µmol.cm⁻².h⁻¹. For example, said hydrogen photocatalytic production may be of from 10 µmol.cm⁻².h⁻¹ to 20 µmol.cm⁻².h⁻¹. As a comparison, with *r* of about 10-25, said values may be, in some embodiments, about 2-3 µmol.cm⁻².h⁻¹.

The temperature range of the substate may be of from 150°C to 225°C, more preferably of from 150°C to 200°C, especially of from 175°C to 200°C, providing polyporphyrin films with the best catalytic effects.

The oxidant is selected to perform the process and may very preferentially be at least one selected from FeCl₃, CuCl₂ and Cu(ClO₄)₂. Most preferred is FeCl₃ as promoting enhanced dehydrogenative coupling over their undesirable chlorination.

Preferably, the step (b) of the process may further be performed with a crosslinking monomer being a fully unsubstituted metalloporphyrin (called metalloporphine), a mono-*meso*-substituted metalloporphyrin and/or an octa-*β*-substituted metalloporphyrin. Indeed, owing to their three to four free *meso* positions, mono-*meso*-substituted metalloporphyrins, fully unsubstituted metalloporphyrins (metalloporphine) and octa-*β*-substituted metalloporphyrins can reticulate three to four polyporphyrin chains. The central metal cation of the fully unsubstituted metalloporphyrin (metalloporphine), a mono-*meso*-substituted metalloporphyrin and/or an octa-*β*-substituted metalloporphyrin being selected from the group consisting of Mg(II), Fe(III), Co(II), Ni(II), Cu(II), Zn(II), Ru(II), Pd(II), Ag(II), Pt(II) and Au(III), or mixtures thereof.

The preferred aromatic groups R are those disclosed above.

The oCVD process can be carried out using commercially available devices or can be a custom-built oCVD reactor. The one skilled in the art may for example refer to WO 2020/099385 and Bengasi et al. to find all required data and devices related to implement the process.

The substrates are of those cited previously. It should be emphasized that the substrate used is heat resistant and is selected according to the process operating temperatures.

The invention also relates to a use of a multiply fused, conjugated, porphyrin polymer film coated on a substrate, said multiply fused, conjugated, porphyrin polymer being of formula (I) of the invention or as obtainable according to the process of the invention, in sensing and/or catalytic devices and/or materials.

Preferably, an hydrogen photocatalytic production, from water splitting, using said multiply fused, conjugated, porphyrin polymer, may be not lower than about 5 µmol.cm⁻².h⁻¹, even not lower than about 9 µmol.cm⁻².h⁻¹, especially not lower than 20 µmol.cm⁻².h⁻¹. For example, said hydrogen photocatalytic production may be of from 10 µmol.cm⁻².h⁻¹ to 20 µmol.cm⁻².h⁻¹.

### Brief description of the drawings

Other features and advantages of the present invention will be readily understood from the following detailed description and drawings among them:
- Figure 1 illustrates the diverse reactions occurring during the oCVD reaction of nickel(II) 5,15-diphenylporphyrin NiDPP with FeCl₃;
- Figure 2 is a schematic view of an example of oxidative chemical vapour deposition (oCVD) reactor for the deposition of conjugated porphyrins using FeCl₃ oxidant;
- Figure 3 is a graph showing the variation of the density of the fused cobalt(II) 5,15-di(4-fluorophenyl) porphyrin thin films as a function of the substrate temperature;
- Figure 4 represents Scanning electron micrographs of the fused cobalt(II) 5,15-di(4-fluorophenyl) porphyrin thin films for different substrate temperatures;
- Figure 5 represents photocatalytic H₂ production rates for the fused cobalt(II) 5,15-di(4-fluorophenyl) porphyrin thin films synthesized at different substrate temperatures;
- Figure 6 shows the variation of the density of the fused copper(II) 5,15-(di-3,5-di-trifluoromethylphenyl)porphyrin thin films as a function of the ratio between FeCl₃ and porphyrin monomer; and
- Figure 7 represents photocatalytic H₂ production rates for the fused copper(II) 5,15-(di-3,5-di-trifluoromethylphenyl)porphyrin thin films as a function of the ratio between FeCl₃ and porphyrin monomer.

### Film Characterizations

The electrical conductivity is measured by depositing the films onto commercial OFET chips. The measurements were done without applying any gate voltage and by recording the current-voltage scans with a two-point probe to extract the conductivity using Ohm's law. The thickness of the films was measured using an Alpha step d-500 profilometer from KLA-Tencor.

The surface density was assessed from the specific mass gain due to the deposition and the area of the coated surface: Surface Density [µg·cm⁻²] = (m₁-m₀)/A, where m₀ and m₁ are the masses of the coated and uncoated substrate, respectively, while A is the coated area estimated using imaged software. Coated microscope glass slides were weighted using a microbalance (Sartorius ME36S) to obtain m₁, then the samples were immersed in a mixture of acetone/ethanol (50:50 %) for 2 min before to be cleaned using Kimtech tissues to remove all the deposited film and obtain m₀. The surface density measurements were repeated on three different samples for each deposition condition to determine the average surface density. The film's density (g.cm⁻³) was calculated from the measured surface density and the thickness of each film.

The static water contact angles (WCA) were evaluated from five different measurements undertaken at different position for each sample using a contact angle measuring instrument (KRÜSS EasyDrop).

The water uptake of the films was evaluated on coated glass substrates by measuring the mass difference of the totally hydrated (m_{wet}) and vacuum-dried films (m_{dry}). Initially, the water from the fully hydrated films was carefully blotted using Kimtech tissues, then dried in a vacuum oven at 70°C for 24 h. The water uptake measurement was repeated five times for each of the prepared thin films and the average value and standard deviation of these measurements are reported.

The optical absorbance was measured in the range of 250-2500 nm using an UV/Vis/NIR spectrophotometer (Perkin Elmer, Lambda 950) with a 150 mm diameter integrating sphere. The absorbance of the as-deposited and acetone rinsed films was measured on the glass substrates. The absorbance of the acetone-soluble phase of the films was measured in quartz cuvettes of 3,5 mL and 1 cm light path. The Helium Ion Microscope (HIM) images were recorded with an Orion Nanofab Instrument from Zeiss. The images were acquired from samples deposited on silicon wafers. Helium ions are generated using a gas field ionization source (GFIS). Within the GFIS a sharp needle having an apex radius of approximately 100 nm is set to a positive high voltage with respect to an extraction electrode accelerating the ions. A landing energy of 30 keV was used, impinging an ion currents between 0,1 and 1 pA and a tilt angle of the substrate holder stage of 20°. The helium ion beam scans the sample surface creating secondary electrons (SE). Contrast in the images is created mainly by composition and topography. Compared to the standard secondary electron microscope (SEM), the HIM allows to probe specimens with a better surface sensitivity and a higher depth of field which makes it very suitable for topographic imaging. Images of the topography and nanomechanical properties (Young modulus) of the samples were acquired using the AM-FM mode of the MFP-3D Infinity Atomic Force Microscopy (AFM) (Asylum Research). All measurements were made under ambient conditions (room temperature and relative humidity of about 50%) and a standard cantilever holder for operation in air was used. Images of a 2×2 µm² area were taken with a resolution of 256×256 pixels at a scan rate of 3 Hz. Cantilevers' spring constants used in this study were about 20-30 N.m⁻¹ (Olympus, AC160TS-R3). The first and second resonant frequencies for AC160TS-R3 cantilevers were about 238 kHz and 1,34 MHz, respectively. A relative calibration method was done to estimate the tip radius using a dedicated reference samples kit provided by Bruker (PFQNM-SMPKIT-12m). The deflection sensitivity and the spring constant of the cantilever was determined using the Thermal Tune method (based on measurement of the thermal noise) and the Sader method. The tip radius was adjusted to obtain the proper value of 2,7 GPa for the polystyrene reference, matching the deformation applied on the sample of interest. To ensure repulsive intermittent contact mode, the amplitude setpoint was chosen as Aₛₑₜₚₒᵢₙₜ/A₀ = ca. 0,7 so that the phase is well fixed at 60°C. The reported average and standard deviation values of Young modulus and roughness (Ra) consider at least 3 images in each sample for reliable results.

Secondary ion mass spectrometry (SIMS) measurements were performed on a CAMECA NanoSIMS 50 using a Cs⁺ primary ion beam with an impact energy of 16 keV and a current of around 1.5 A on sample surface. The masses studied simultaneously in multicollection mode were ¹²C, ¹⁴N, ³⁵Cl and ⁵⁶Fe, ¹²O. Images were acquired at a size of 40×40 µm and 256×256 pixels. Secondary electron (SE) images were recorded using the same SIMS instrument. Atmospheric-pressure laser desorption/ionization coupled with a high-resolution mass spectrometer (AP-LDI-HRMS) was used for the characterization and identification of the oligomers produced by the oCVD reaction of porphyrin monomers with FeCl₃.

HRMS analyses were performed with an LTQ/Orbitrap Elite^{™} Hybrid Linear Ion Trap-Orbitrap Mass Spectrometer from Thermo Scientific (San Jose, CA) coupled with an AP-LDI (ng) UHR source from MassTech Inc (Columbia, MA) with a 355 nm Nd:YAG laser. The as-deposited polyporphyrins thin films were directly probed, without any matrix deposition, by the laser following a spiral motion during 30 seconds per sample. An in-source decay (ISD) of 70 V was applied to the samples in order to prevent any formation of non-covalent polyporphyrins clusters that could interfere with the distribution of the polyporphyrins oligomers. A maximum injection time of 800 ms and a resolving power of 240,000 at m/z 400 within the normal mass range (m/z 300-2000) and the high mass range (m/z 1800-4000) were employed for the HRMS analyses.

X-ray photoelectron spectroscopy (XPS) analyses were performed on a Kratos Axis Ultra DLD instrument using a monochromatic Al Kα X- ray source (Zu = 1486,6 eV) at a power of 105 W. Charge calibration was accomplished by fixing the binding energy of carbon (C 1s) to 285,0 eV.

The photocatalytic hydrogen evolution test is performed in a 250 mL glass reactor, using 200 mL of an aqueous solution with 10% of a sacrificial agent, such as alcohols, amines, carboxylic acids, especially EDTA. At the beginning of the test, N₂ is bubbled for 10 min to remove the oxygen or adsorbed gases from the reaction media. After this, the reactor is illuminated with a solar simulator integrated with a Xe lamp of 450 W. The H₂ produced is analyzed in a Thermo Scientific gas chromatograph coupled with a thermal conductivity detector every 30 min during 3 h.

### Example1 : nickel(II) 5,15-diphenylporphyrin (NiDPP)

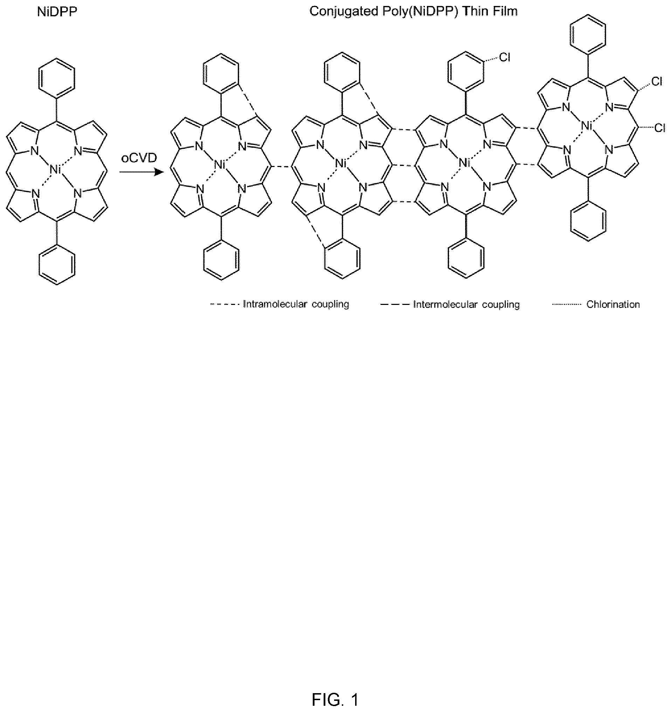

A series of fused porphyrin tapes thin films was prepared from the oCVD reaction of nickel(II) 5,15-diphenylporphyrin (NiDPP) and iron(III) chloride (FeCl₃), commercially available. NiDPP is selected due to its proven ability to form multiply-fused porphyrin tapes in oCVD (see G. Bengasi; K. Baba; O. Back; G. Frache; K. Heinze; N. D. Boscher; "Reactivity of Nickel(II) Porphyrins in oCVD Processes- Polymerisation, Intramolecular Cyclisation and Chlorination"; Chem. Eur. J., 2019, 25, 8313-8320), while FeCl₃ was previously demonstrated as a better choice to promote the dehydrogenative coupling of porphyrins over their undesired chlorination. Fig.1 illustrates the diverse reactions occurring during the oCVD reaction of nickel(II) 5,15-diphenylporphyrin NiDPP with FeCl₃. Alongside the targeted dehydrogenative coupling of NiDPP, which can form singly, doubly or triply-fused porphyrin tapes, chlorination and π-extension via ring fusion can occur.

### Example 2: Cobalt(II) 5,15-di(4-fluorophenyl) porphyrin

Fused cobalt(II) 5,15-di(4-fluorophenyl) porphyrin thin films were deposited on silicon wafers, microscope glass slides and conductive FTO-coated substrates from the oxidative chemical vapour deposition (oCVD) reaction of cobalt(II) 5,15-di(4-fluorophenyl)porphyrin (CoD4FPP) with FeCl₃.

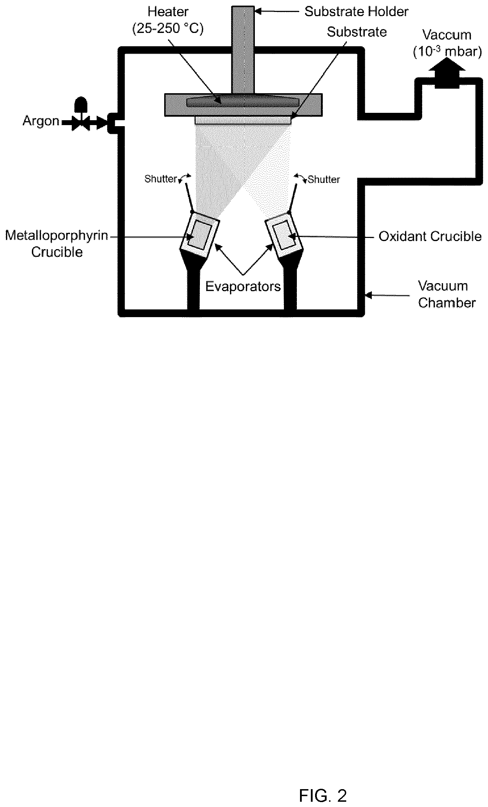

The oCVD reaction was performed in a stainless-steel reaction chamber equipped with two crucibles used to simultaneously sublimate the porphyrin and the oxidant, as shown in Fig.2. The crucibles were loaded with 10 mg of CoD4FPP and 500 mg of FeCl₃, *r* = 50, and heated to 250°C and 150°C, respectively.

The oCVD reactor was also equipped with a dry scroll pump (Varian) and a turbomolecular pump (Leybold) to achieve high vacuum. A butterfly type throttling valve (VAT) and a microleak valve fed with argon (Air Liquide, 99,999 %) were used to maintain the pressure to 10⁻³ mbar for the preparation of the thin films. Under these conditions, FeCl₃, was sublimated in ca. 50-fold excess with respect to the porphyrin monomer. Taking advantage of the solvent-free conditions of the oCVD method, different substrate temperatures, also called reaction temperature, were investigated. In particular, the substrate temperature was varied from room temperature (ca. 25°C) to 200°C.

Irrespective of the substrate temperature, the oCVD reaction of CoD4FPP and FeCl₃ yields the formation of macroscopically smooth and uniform thin films with an electrical conductivity in the range of 10⁻³ to 10⁻¹S.cm⁻¹. The oCVD thin films exhibit colorations that strongly differ from the light orange color of the reference thin film prepared from the sublimation of CoD4FPP without oxidant. Such a color change is indicative of the formation of fused porphyrin tapes. Nevertheless, the discrepancy between the greenish coloration of the oCVD thin films elaborated at 25°C and the dark brownish color of the oCVD thin films obtained at 200°C hints on a difference in the deposition rate and/or in the reactivity, i.e. different reaction kinetics between the dehydrogenative coupling, chlorination and π-extension via ring fusion of the porphyrins. Interestingly, ultraviolet-visible-near infrared (UV/Vis/NIR) spectrophotometry evidenced a slightly more pronounced NIR absorption with the increase of the substrate temperatures, suggesting a higher degree of conjugation for higher substrate temperatures. Atmospheric-pressure laser desorption/ionization high-resolution mass spectrometer (AP-LDI-HRMS) analysis confirms the successful oxidative polymerization of CoD4FPP, i.e. intermolecular dehydrogenative coupling, irrespective of the substrate temperature. In addition, AP-LDI-HRMS also evidences chlorination (+Cl -H)ₙ and intramolecular cyclisation (-H₂)ₙ reactions known to occur during the oCVD of porphyrins. Intramolecular cyclisation, i.e. intramolecular dehydrogenative coupling, occurs between the free *ortho* position of the 4-fluorophenyl substituent and *beta* position of the porphyrin macrocycle. Although AP-LDI-HRMS analysis does not provide an exhaustive view into the mass distribution and that the intensities related to the different species detected are not fully related to their abundance, the high mass resolution (up to 240,000 at m/z 400) and the high mass accuracy of the technique, around 3 ppm, enables the draw of informative trends. In particular, the analysis of the dimeric and trimeric regions of the spectra reveals the incorporation of a greater number of chlorine (+Cl -H)ₙ upon increase of the substrate temperature from 25°C to 100°C. Surprisingly, a lesser amount of chlorine is integrated to the polymer structure upon further increase of the temperature to 200°C. On the other hand, the number of unsaturation in the molecule (-2H)ₙ, related to both intermolecular and intramolecular dehydrogenative coupling, is constantly increasing with an increase of the substrate temperature.

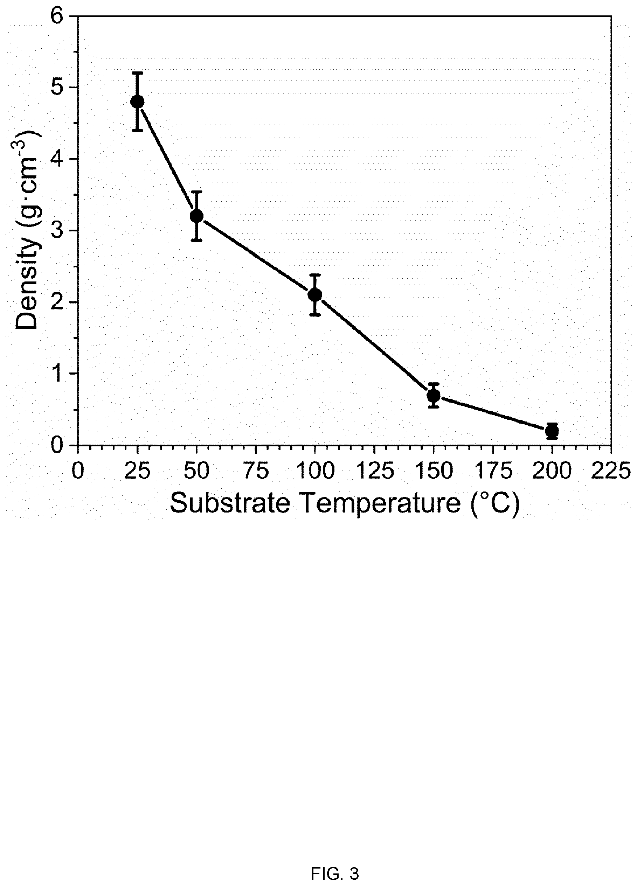

Contact profilometry measurements highlighted strong discrepancies between the thicknesses of the fused cobalt(II) 5,15-di(4-fluorophenyl) porphyrin films. Indeed, while an identical amount of reactants is delivered to the substrate, thickness of the formed thin films varied from over 1 order of magnitude. In particular, thickness increased from 50 nm to 1,5 µm upon increase of the substrate temperature from 25°C to 200°C. Surprisingly, in spite of the large growth rate discrepancies observed for the oCVD thin films, their weight per unit area was shown constant for all the investigated substrate temperatures, an almost identical to the weight per unit area of the reference thin film prepared from the sublimation of CoD4FPP without FeCl₃. Such observation is consistent with the sublimation of identical amounts of porphyrin during the preparation of the thin films, but suggests strong variations in the density of the films. Indeed, the calculated density is shown to drastically decrease with increase of the substrate temperature. As shown in Fig. 3, the density calculated for the thin film elaborated at 200°C (ca. 0,21 g·cm⁻³) being over 20 times smaller than the one calculated for the thin film elaborated at 25°C (ca. 4,8 g·cm⁻³), and evidences the formation of highly porous thin films when employing large excess of oxidant and high substrate temperatures.

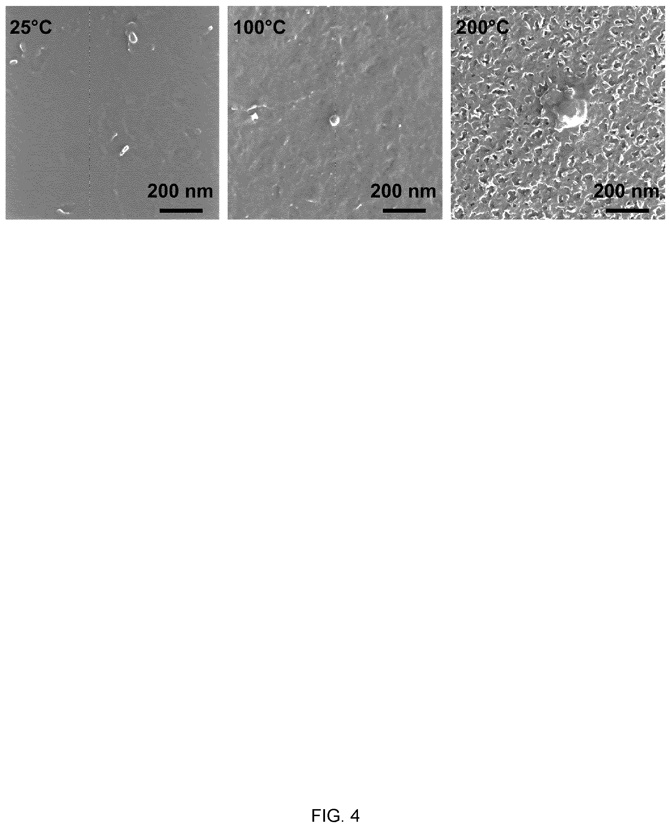

The decrease of the film's density is coupled to a decrease of the Young's modulus. In particular, the Young modulus value decreases from 5 ± 1 GPa for the fused cobalt(II) 5,15-di(4-fluorophenyl) porphyrin films prepared at 25°C to 1,5 ± 0,5 GPa for fused cobalt(II) 5,15-di(4-fluorophenyl) porphyrin films prepared at 200°C. Helium ion microscopy illustrated the formation of mesoporous thin films at high substrate temperatures, while denser layers are formed below 100°C, as depicted in Fig. 4. Fig. 4 for 200°C shows some pores which are exhibiting various forms.

The low density and mesoporous structure of the fused cobalt(II) 5,15-di(4-fluorophenyl) porphyrin films elaborated at the highest temperatures, is responsible of a greater water uptake for these samples. Specifically, water uptake between 0,8 mg/mm³ to 1,0 mg/mm³ are measured for the films elaborated at temperatures comprised between 150°C and 200°C. It is worth noting that irrespective of the deposition condition, the water contact angle of the as-deposited fused cobalt(II) 5,15-di(4-fluorophenyl) porphyrin films remained fairly constant to 90°.

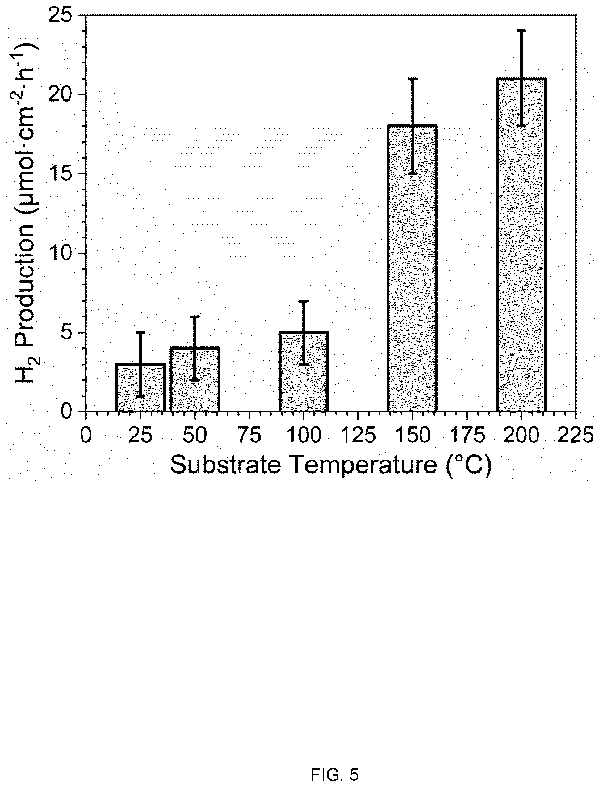

Upon exposure to visible light irradiation (Xenon lamp, 450 W), the fused cobalt(II) 5,15-di(4-fluorophenyl) porphyrin films evolved hydrogen when immersed in a water solution containing 10% of ethylenediaminetetraacetic acid (EDTA). Interestingly, the photocatalytic catalytic performances of the fused cobalt(II) 5,15-di(4-fluorophenyl) porphyrin thin films directly correlated with their density and superior hydrogen production rates were achieved for the thin films prepared at 200°C (Fig.5). The formation of fused porphyrin thin films with highly porous structure, characterized by a density lower than 1 g.cm⁻³, enabled to maximize the yield of the photocatalytic reaction.

### Example 3: Copper(II) 5,15-(di-3,5-di-trifluoromethylphenyl)porphyrin

Fused copper(II) 5,15-(di-3,5-di-trifluoromethylphenyl)porphyrin films were deposited on silicon wafers, microscope glass slides and conductive FTO-coated substrates from the oxidative chemical vapour deposition (oCVD) reaction of copper(II) 5,15-(di-3,5-di-trifluoromethylphenyl)porphyrin (CuDDTFMPP) with FeCl₃.

The oCVD reaction was performed with the reaction chamber described in Example 2.

The pressure was 10⁻³ mbar and the substrate temperature was 200°C for all the deposition experiment duration, i.e. 30 min. The CuDDTFMPP was heated at 270°C, while the FeCl₃ crucible temperature was varied from 80°C to 200°C in order to investigate different oxidant to porphyrin ratios, i.e. *r* of from 1 to 200.

The oCVD thin films exhibit colorations that strongly differ from the oxidant to porphyrin ratio. The thin film elaborated at the lowest *r,* of from 1 to 10, exhibit an orange coloration close to the one of the reference thin film prepared from CuDDTFMPP without oxidant, which suggests a low degree of polymerization for low oxidant to porphyrin ratios. This is not surprising as the high sticking coefficient of porphyrins implies that they can deposit on the substrate and form a thin film even if non-polymerised.

For *r* > 10, the CuDDTFMPP-based films exhibit a green coloration that gets darker upon further increase of the oxidant to porphyrin ratio. Such a color change is indicative of the intermolecular dehydrogenative coupling of the CuDDTFMPP porphyrins, such as confirmed by AP-LDI-HRMS. AP-LDI-HRMS also evidenced the chlorination and π-extension via ring fusion of the porphyrin tapes, which both get more pronounced upon increase of the oxidant to porphyrin ratio. As a consequence, the electrical conductivity of the CuDDTFMPP-based films is strongly affected by the oxidant to porphyrin ratio and varies for example from 10⁻⁹ to 10⁻⁴ S.cm⁻¹ for the films elaborated with an *r* of 1 and 50, respectively.

Contact profilometry measurements highlighted strong discrepancies between the thicknesses of the CuDDTFMPP-based films. In particular, thickness increased from 50 nm to 2,0 µm upon increase of the oxidant to porphyrin ratios from 1 to 200, the highest thickness values of the range is for high *r.*

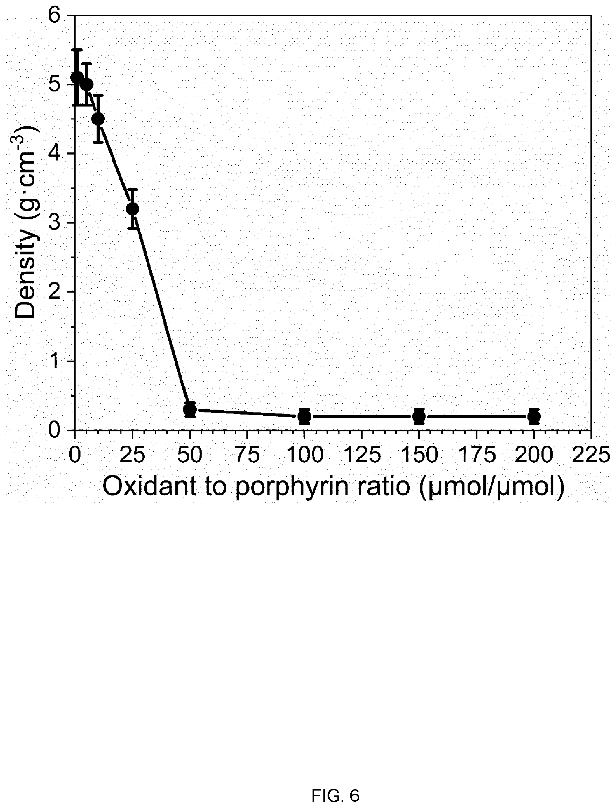

While an identical amount of porphyrin is delivered to the substrate, such as confirmed by the almost identical weight per unit area of the thin films prepared from the different *r* investigated, these thickness discrepancies evidence strong variation of the CuDDTFMPP-based films density, as illustrated in Fig. 6. The density calculated for the thin film elaborated at *r* greater than 50 (ca. 0,20 g·cm⁻³) being over 25 times smaller than the one calculated for the thin film elaborated at oxidant to porphyrin ratios lower than 5 (ca. 5 g·cm⁻³) and suggest the formation of highly porous thin films when employing large excess of oxidant and high substrate temperatures. The decrease of the film's density is coupled to a decrease of the Young's modulus, which decreases from 5 ± 1 GPa to 2 ± 0.5 GPa upon increase of the oxidant to porphyrin ratios from 1 to ratios higher to 50.

Owing to the presence of the trifluoromethyl groups, the water contact angle of the CuDDTFMPP-based films is slightly hydrophobic with values comprise between 90° and 110°. Nevertheless, the CuDDTFMPP-based films are demonstrated to uptake water with an extend that depend on the used oxidant to porphyrin ratios. The highest water uptakes (up to 1,0 mg/mm³) being obtained for films prepared from oxidant to porphyrin ratios of from 50 to 100. For oxidant to porphyrin ratios superior to 150, delamination of the films occurs upon immersion in water.

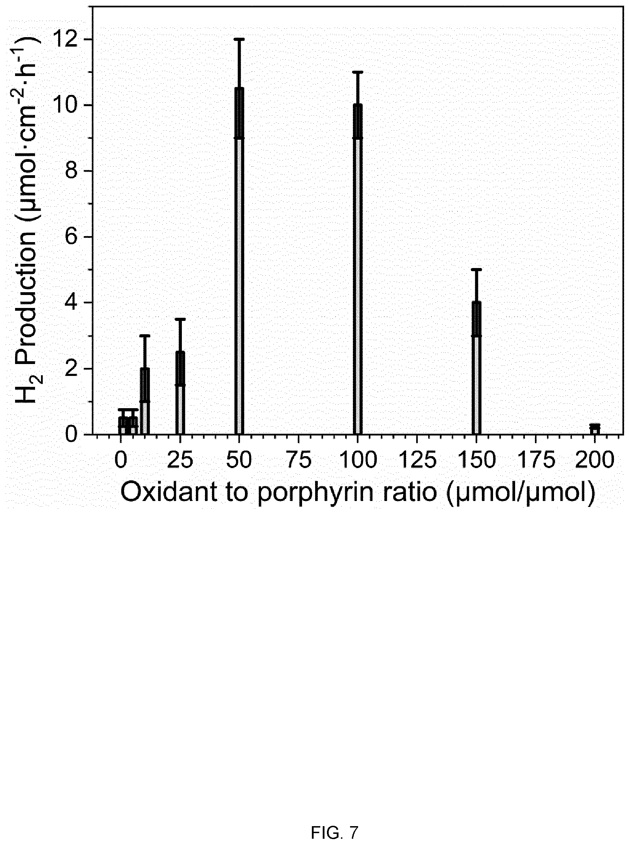

Upon exposure to visible light irradiation (Xenon lamp, 450 W), the CuDDTFMPP-based films immersed in a water solution containing 10% of ethylenediaminetetraacetic acid (EDTA) exhibited hydrogen production rates that strongly depend on the oxidant to porphyrin ratio. Interestingly, as seen in Fig. 7, the photocatalytic catalytic activity of the CuDDTFMPP-based thin films reached a maximum for *r* of from 50 to 100. In spite of a similar degree of polymerization, CuDDTFMPP-based films elaborated for *r* of 25 exhibited a much lower photocatalytic activity, which is attributed to the lower specific surface area of this sample.

For *r* > 150, a collapse of the photocatalytic activity is observed. Such a decrease of the photocatalytic properties is attributed to the cracking and decohesion of the CuDDTFMPP-based thin films related to the excess of oxidant and oxidant by-products trapped into the thin films and dissolved upon immersion in aqueous media.

## Claims

1. A multiply fused, conjugated, porphyrin polymer film coated on a substrate, said multiply fused porphyrin polymer being of formula (I) 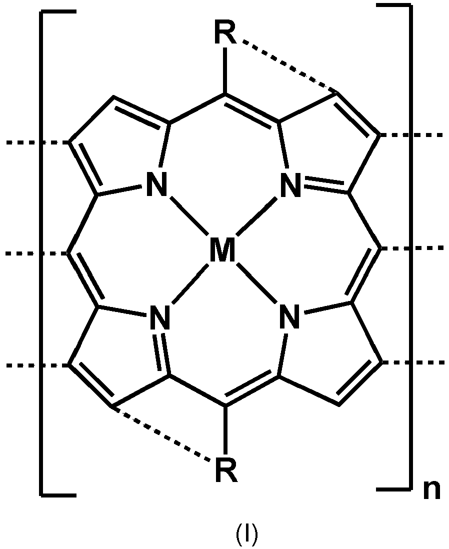 wherein:
"n" is an integer greater than 1;
the porphyrin monomer repeating units are di-meso-substituted porphyrins;
M is a metal cation selected from the group consisting of Mg(II), Fe(III), Co(II), Ni(II), Cu(II), Zn(II), Ru(II), Pd(II), Ag(II), Pt(II) and Au(III), or mixtures thereof;
the porphyrin units are multiply fused, including doubly-fused and/or triply-fused;
each substituent R attached to the *meso* position of the porphyrin monomer is an aromatic group presenting at least one free *ortho* position among;
at least one of the two free *ortho* positions of the aromatic substituent R is fused to the *β* position of the porphyrin monomer,
**characterized in that** said porphyrin polymer film is a porous porphyrin polymer film with mean pore diameters, determined by SEM, within the range of from 2 nm to 100 nm, and exhibiting a density, calculated as disclosed in the description, not greater than 2 g/cm³.

2. The multiply fused, conjugated, polymer porphyrin film coated on a substrate according to claim 1, wherein the porphyrin monomer units are multiply fused, including doubly-fused, i.e *β-meso, meso-β,* and triply-fused, i.e *β*-*β*, *meso-meso*, *β-β.*

3. The multiply fused, conjugated, polymer porphyrin film coated on a substrate according to claim 1 or 2, wherein M is selected from Co(II) and Cu(II), or mixture thereof.

4. The multiply fused, conjugated, polymer porphyrin film coated on a substrate according to any of claims 1 to 3, wherein the mean pore diameters is within the range of from 5 nm to 80 nm, and, independently, the density is of from 0,20 g/cm³ to 1,5 g/cm³, more preferably of from 0,20 g/cm³ to 1,2 g/cm³, most preferably of from 0,25 g/cm³ to 1,0 g/cm³.

5. The multiply fused, conjugated, polymer porphyrin film coated on a substrate according to any of claims 1 to 4, wherein the n value of the porphyrin repeating units is of from 2 to 50, more preferably of from 10 to 50, most preferably, of from 10 to 40.

6. The multiply fused, conjugated, polymer porphyrin film coated on a substrate according to any of claims 1 to 5, presenting at least one of the following characteristics:
- a thickness of from 500 nm to 10 µm, more preferably of from 500 nm to 5 µm, especially of from 500 nm to 2 µm;
- an electrical conductivity of from 10⁻⁴ S/cm to 10 S/cm;
- a Young modulus of from 1 GPa to 3 GPa; and
- a water uptake of from 0,5 mg/mm³ of H₂O to 1,0 mg/mm³ of H₂O, more preferably of from 0,8 mg/mm³ of H₂O to 1,0 mg/mm³ of H₂O.

7. The multiply fused, conjugated, polymer porphyrin film coated on a substrate according to any of claims 1 to 6, wherein the aromatic group R is a 6-membered aromatic ring structure selected from the group consisting of phenyl, 4-pyridyl, 3-pyridyl, 4-methylphenyl (p-tolyl), 3,5-di-tert-butylphenyl, 4-hydroxyphenyl, 4-carboxyphenyl, 4-aminophenyl, 3-cyanophenyl, 4-trifluoromethyl-phenyl, 4-fluorophenyl, 3,5-bis(trifluoromethyl)phenyl, 4-chlorophenyl, 4-bromophenyl, naphthalene, anthracene, phenanthrene and pyrene, or mixtures thereof.

8. A process for forming, on a substrate, a multiply fused, conjugated, porphyrin polymer of formula (I) according any of claims 1 to 7, the method comprising the steps of:
(a) providing a substrate in a vacuum chamber,
(b) performing on said substrate an oxidative chemical vapour deposition reaction with a predetermined quantity of at least one oxidant selected from FeCl₃, CuCl₂ and Cu(ClO₄)₂, and a predetermined quantity of at least one porphyrin monomer of general formula (II): 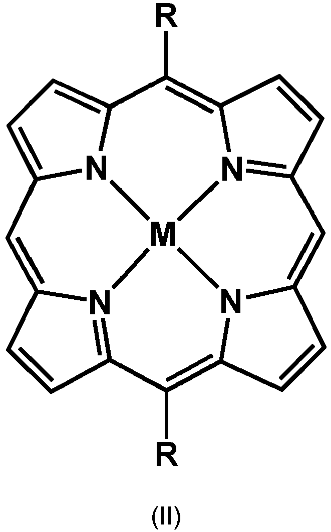
wherein
- M is a metal cation selected from the group consisting of Mg(II), Fe(III), Co(II), Ni(II), Cu(II), Zn(II), Ru(II), Pd(II), Ag(II), Pt(II) and Au(III), or mixtures thereof;
- each substituent R attached to the meso position of the porphyrin monomer is an aromatic group presenting at least one free *ortho* position;
**characterized in that** step (b) is carried at a substrate temperature range of from 150°C to 250°C, and **in that** the ratio (*r*) of the at least one oxidant molar quantity/at least one porphyrin monomer molar quantity is within a range of from 35 to 150.

9. The process according to claim 8, wherein the *r* range is of from 45 to 125, most preferably of from 50 to 100.

10. The process according to claim 8 or 9, wherein the temperature range of the substate is of from 150°C to 225°C, more preferably of from 150°C to 200°C, especially of from 175°C to 200°C.

11. The process according to any of claims 8 to 10, wherein step b) is further performed with a crosslinking monomer being a mono-meso-substituted metalloporphyrin and/or an unsubstituted metalloporphyrine and/or an octa-*β-*substituted metalloporphyrin which central metal cation is selected from the group consisting of Mg(II), Fe(III), Co(II), Ni(II), Cu(II), Zn(II), Ru(II), Pd(II), Ag(II), Pt(II) and Au(III), or mixtures thereof.

12. A use of a multiply fused, conjugated, porphyrin polymer film coated on a substrate, said multiply fused porphyrin polymer being of formula (I) any of claims 1-7 or as obtainable according to the process of any of claims 8-11, in sensing and/or catalytic devices and/or materials.

13. The use according to claim 12, wherein an hydrogen photocatalytic production, from water splitting, with said multiply fused, conjugated, porphyrin polymer, is not lower than about 5 µmol.cm⁻².h⁻¹, even not lower than about 9 µmol.cm⁻².h⁻¹, especially not lower than 20 µmol.cm⁻².h⁻¹.

## Patentansprüche

1. Ein mehrfach verschmolzener, konjugierter Porphyrinpolymerfilm, der auf ein Substrat aufgetragen ist, wobei das mehrfach verschmolzene Porphyrinpolymer die Formel (I) hat 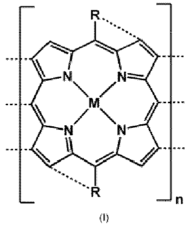 worin:
"n" eine ganze Zahl größer als 1 ist;
die sich wiederholenden Porphyrin-Monomer-Einheiten di-meso-substituierte Porphyrine sind;
M ein Metallkation ist, ausgewählt aus der Gruppe bestehend aus Mg(II), Fe(III), Co(II), Ni(II), Cu(II), Zn(II), Rui), Pd(II), Ag(II), Pt(I) und Au(III) oder Mischungen davon;
die Porphyrineinheiten mehrfach verschmolzen sind, einschließlich zweifach verschmolzen und/oder dreifach verschmolzen;
jeder Substituent R, der an die *meso*-Position des Porphyrinmonomers gebunden ist, eine aromatische Gruppe ist, die mindestens eine freie *ortho*-Position aufweist, darunter;
mindestens eine der beiden freien *ortho*-Positionen des aromatischen Substituenten R mit der *β*-Position des Porphyrinmonomers verschmolzen ist,
**dadurch gekennzeichnet, dass** der Porphyrinpolymerfilm ein poröser Porphyrinpolymerfilm mit mittleren Porendurchmessern, die mittels SEM bestimmt wurden, im Bereich von 2 nm bis 100 nm ist und eine Dichte, berechnet wie in der Beschreibung offenbart, von nicht mehr als 2 g/cm³ aufweist.

2. Der mehrfach verschmolzene, konjugierte, polymere Porphyrinfilm, der auf ein Substrat gemäß Anspruch 1 aufgetragen ist, wobei die Porphyrinmonomereinheiten mehrfach verschmolzen sind, einschließlich doppelt verschmolzen, d.h. *β-meso, meso-β,* und dreifach verschmolzen, d.h. *β-β, meso-meso, β-β.*

3. Der mehrfach verschmolzene, konjugierte, polymere Porphyrinfilm, der auf ein Substrat gemäß Anspruch 1 oder 2 aufgebracht ist, wobei M aus Co(II) und Cu(II) oder einer Mischung davon ausgewählt ist.

4. Der mehrfach verschmolzene, konjugierte Polymerporphyrinfilm, der gemäß einem der Ansprüche 1 bis 3 auf ein Substrat aufgebracht ist, wobei die mittleren Porendurchmesser im Bereich von 5 nm bis 80 nm liegen und unabhängig davon die Dichte von 0,20 g/cm³ bis 1,5 g/cm³, bevorzugter von 0,20 g/cm³ bis 1,2 g/cm³, am meisten bevorzugt von 0,25 g/cm³ bis 1,0 g/cm³ beträgt.

5. Der mehrfach verschmolzene, konjugierte, polymere Porphyrinfilm, der gemäß einem der Ansprüche 1 bis 4 auf ein Substrat aufgebracht ist, wobei der n-Wert der sich wiederholenden Porphyrineinheiten 2 bis 50, bevorzugter 10 bis 50, am meisten bevorzugt 10 bis 40 beträgt.

6. Der mehrfach verschmolzene, konjugierte, polymere Porphyrinfilm, der gemäß einem der Ansprüche 1 bis 5 auf ein Substrat aufgebracht ist, weist mindestens eine der folgenden Eigenschaften auf:
eine Dicke von 500 nm bis 10 µm, bevorzugter von 500 nm bis 5 um, insbesondere von 500 nm bis 2 µm;
- anelektrische Leitfähigkeit von 10⁻⁴S/cm bis 10 S/cm;
- einen Young-Modul von 1 GPa bis 3 GPa; und
- eine Wasseraufnahme von 0,5 mg/mm³ H₂O bis 1,0 mg/mm³ H₂O, besonders bevorzugt von 0,8 mg/mm³ H₂O bis 1,0 mg/mm³ H₂O.

7. Der Der mehrfach verschmolzene, konjugierte, polymere Porphyrinfilm, der nach einem der Ansprüche 1 bis 6 auf ein Substrat aufgebracht ist, wobei die aromatische Gruppe R eine 6-teilige aromatische Ringstruktur ist, ausgewählt aus der Gruppe bestehend aus Phenyl, 4-Pyridyl, 3-Pyridyl, 4-Methylphenyl (p-Tolyl), 3,5-Di-tert-butylphenyl, 4-Hydroxyphenyl, 4-Carboxyphenyl, 4-Aminophenyl, 3-Cyanophenyl, 4-Trifluormethylphenyl, 4-Fluorphenyl, 3,5-Bis(trifluormethyl)phenyl, 4-Chlorphenyl, 4-Bromphenyl, Naphthalin, Anthracen, Phenanthren und Pyren oder Mischungen davon.

8. Ein Verfahren zur Bildung eines mehrfach kondensierten, konjugierten Porphyrin-Polymers der Formel (I) nach einem der Ansprüche 1 bis 7 auf einem Substrat, wobei das Verfahren die folgenden Schritte umfasst:
(a) Bereitstellen eines Substrats in einer Vakuumkammer,
(b) Durchführen einer oxidativen chemischen Dampfabscheidungsreaktion auf dem Substrat mit einer vorbestimmten Menge mindestens eines Oxidationsmittels, ausgewählt aus FeCl₃, CuCl₂ und Cu(ClO₄)₂, und einer vorbestimmten Menge mindestens eines Porphyrinmonomers der allgemeinen Formel (II): 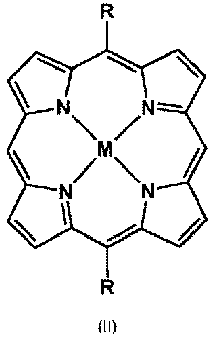
worin
- M ein Metallkation ist, das aus der Gruppe ausgewählt ist, die aus Mg(II), Fe(III), Co(II), Ni(II), Cu(II), Zn(II), Ru(II), Pd(II), Ag(II), Pt(II) und Au(III) oder Mischungen davon besteht;
- jeder Substituent R, der an die *meso*-Position des Porphyrin-Monomers gebunden ist, eine aromatische Gruppe ist, die mindestens eine freie *ortho*-Position aufweist;
**dadurch gekennzeichnet, dass** die Stufe (b) bei einer Substrattemperatur im Bereich von 150°C bis 250°C durchgeführt wird und dass das Verhältnis (r) der molaren Menge des mindestens einen Oxidationsmittels zur molaren Menge des mindestens einen Porphyrinmonomers im Bereich von 35 bis 150 liegt.

9. Verfahren nach Anspruch 8, wobei der Bereich von r zwischen 45 und 125 liegt, am meisten bevorzugt zwischen 50 und 100.

10. Das Verfahren nach Anspruch 8 oder 9, wobei der Temperaturbereich des Substats von 150°C bis 225°C, bevorzugter von 150°C bis 200°C, besonders bevorzugt von 175°C bis 200°C beträgt.

11. Das Verfahren nach einem der Ansprüche 8 bis 10, wobei Schritt b) ferner mit einem Vernetzungsmonomer durchgeführt wird, das ein mono-meso-substituiertes Metalloporphyrin und/oder ein unsubstituiertes Metalloporphyrin und/oder ein octa-6-substituiertes Metalloporphyrin ist, dessen zentrales Metallkation ausgewählt ist aus der Gruppe bestehend aus Mg(II), Fe(III), Co(II), Ni(II), Cu(II), Zn(II), Ru(II), Pa(II), Ag(II), Pt(II) und Au(II), oder Mischungen davon.

12. Verwendung eines mehrfach verschmolzenen, konjugierten Porphyrinpolymerfilms, der auf ein Substrat aufgebracht ist, wobei das mehrfach verschmolzene Porphyrinpolymer die Formel (I) eines der Ansprüche 1-7 aufweist oder gemäß dem Verfahren eines der Ansprüche 8-11 erhältlich ist, in sensorischen und/oder katalytischen Vorrichtungen und/oder Materialien.

13. Die Verwendung nach Anspruch 12, wobei die photokatalytische Wasserstoffproduktion aus der Wasserspaltung mit dem mehrfach verschmolzenen, konjugierten Porphyrinpolymer nicht weniger als etwa 5 µmol.cm⁻².h⁻¹, sogar nicht weniger als etwa 9 µmol.cm⁻².h⁻¹, insbesondere nicht weniger als 20 µmol.cm⁻².h⁻¹ beträgt.

## Revendications

1. Un film polymère de porphyrine multi-fusionnée revêtu sur un substrat, ledit film polymère de porphyrine multi-fusionnée est de formule (I) 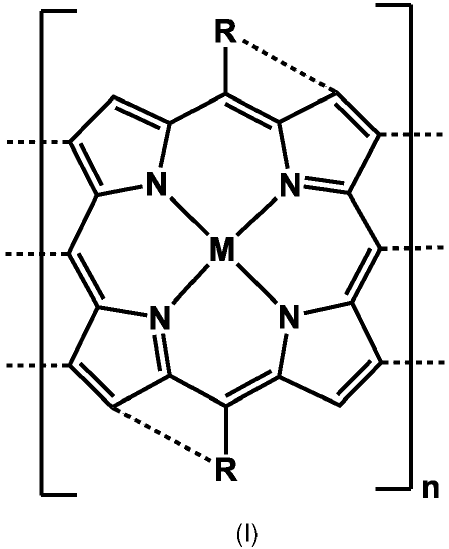 dans laquelle:
"n" est un entier supérieur à 1 ;
les unités de répétition de monomères porphyrine sont des porphyrines di-*méso*-substituées ;
M est un cation métallique choisi dans le groupe constitué par Mg(II), Fe(III), Co(II), Ni(II), Cu(II), Zn(II), Ru(II), Pd(II), Ag(II), Pt(II) et Au(III), ou leurs mélanges ;
les unités de porphyrine sont multi-fusionnées, comprenant les doublement fusionnées et/ou les triplement fusionnées ;
chaque substituant R attaché à la position *méso* du monomère porphyrine est un groupe aromatique présentant au moins une position *ortho* libre ;
au moins l'une des deux positions *ortho* libres du substituant aromatique est fusionné à la position *β* du monomère porphyrine ;
**caractérisé en ce que** ledit film polymère de porphyrine est un film polymère de porphyrine poreux avec des diamètres de pores moyens, déterminés par MEB, dans la gamme de 2 nm à 100 nm, et **en ce qu'**il présente une densité, calculée tel que présenté dans la description, inférieure ou égale à 2 g/cm³.

2. Le film polymère de porphyrine multi-fusionnée revêtu sur un substrat selon la revendication 1, dans lequel les unités monomères de porphyrine sont multi-fusionnées, comprenant les doublement fusionnées, i.e *β-méso, méso-β,* et les triplement fusionnées, i.e *β*-*β*, *méso-méso, β-β.*

3. Le film polymère de porphyrine multi-fusionnée revêtu sur un substrat selon la revendication 1 ou 2, dans lequel M est choisi parmi Co(II) et Cu(II), ou leur mélange.

4. Le film polymère de porphyrine multi-fusionnée revêtu sur un substrat selon l'une des revendications 1 à 3, dans lequel les diamètres de pores moyens est situé dans la gamme de 5 nm à 80 nm, et, indépendamment, la densité est de 0,20 g/cm³ à 1,5 g/cm³, plus préférablement de 0,20 g/cm³ à 1,2 g/cm³, de façon la plus préféré de 0,25 g/cm³ à 1,0 g/cm³.

5. Le film polymère de porphyrine multi-fusionnée revêtu sur un substrat selon l'une des revendications 1 à 4, dans lequel la valeur de n des unités de répétition porphyrine est de 2 à 50, plus préférablement de 10 à 50, de façon la plus préférée de 10 à 40.

6. Le film polymère de porphyrine multi-fusionnée revêtu sur un substrat selon l'une des revendications 1 à 5, présentant au moins l'une des caractéristiques suivante :
- une épaisseur de 500 nm à 10 µm, plus préférablement de 500 nm à 5 µm, en particulier de 500 nm à 2 µm ;
- une conductivité électrique de 10⁻⁴ S/cm à 10 S/cm ;
- un module de Young de 1 GPa à 3 GPa ; et
- une absorption d'eau de 0,5 mg/mm³ de H2O à 1,0 mg/mm³ de H₂O, plus préférablement de 0,8 mg/mm³ de H₂O à 1,0 mg/mm³ de H₂O.

7. Le film polymère de porphyrine multi-fusionnée revêtu sur un substrat selon l'une des revendications 1 à 6, dans lequel le groupe aromatique R est une structure de type cycle aromatique à 6 membres choisie dans le groupe constitué par les phényle, 4-pyridyle, 3-pyridyle, 4-méthylphényle (p-tolyl), 3,5-di-tert-butylphényle, 4-hydroxyphényle, 4-carboxyphényle, 4-aminophényle, 3-cyanophényle, 4-trifluorométhyl-phényle, 4-fluorophényle, 3,5-bis(trifluorométhyl)phényle, 4-chlorophényle, 4-bromophényle, naphtalène, anthracène, phénantrène et pyrène, ou leurs mélanges.

8. Un procédé de préparation, sur un substrat, d'un film polymère de porphyrine multi-fusionnée de formule (I) selon l'une des revendications 1 à 7, le procédé comprenant les étapes de :
(a) fournir un substrat dans une chambre à vide,
(b) mettre en oeuvre sur ledit substrat une réaction de dépôt chimique en phase vapeur oxydante avec une quantité prédéterminée d'un oxydant et une quantité prédéterminée d'au moins un monomère de porphyrine de formule générale (II) : 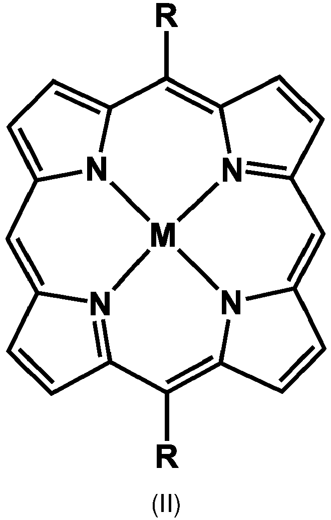 dans laquelle:
- M est un cation métallique choisi dans le groupe constitué par Mg(II), Fe(III), Co(II), Ni(II), Cu(II), Zn(II), Ru(II), Pd(II), Ag(II), Pt(II) et Au(III), ou leurs mélanges ;
- chaque substituant R attaché à la position *méso* du monomère porphyrine est un groupe aromatique présentant au moins une position *ortho* libre ;
**caractérisé en ce que** l'étape (b) est effectuée à une gamme en températures de 150°C à 250°C, et **en ce que** le rapport (*r*) de la quantité de l'au moins un oxydant/la quantité de l'au moins un monomère porphyrine est dans la gamme de 35 à 150.

9. Le procédé selon la revendication 8, dans lequel la gamme de *r* est 45 à 125, de façon la plus préférée de 50 to 100.

10. Le procédé selon la revendication 8 ou 9, dans lequel la gamme en températures du substrat est de 150°C à 225°C, plus préférablement de 150°C à 200°C, en particulier de 175°C à 200°C.

11. Le procédé selon l'une des revendications 8 à 10, dans lequel l'étape (b) est en outre effectuée par un monomère de réticulation étant une métalloporphyrine mono-*méso*-substituée et/ou une métalloporphyrine non substituée et/ou une métalloporphyrine octa-*β*-substituée dont le cation métallique central est choisi dans le groupe constitué par Mg(II), Fe(III), Co(II), Ni(II), Cu(II), Zn(II), Ru(II), Pd(II), Ag(II), Pt(II) et Au(III), ou leurs mélanges.

12. Une utilisation d'un film polymère de porphyrine multi-fusionnée revêtu sur un substrat, ledit film polymère de porphyrine multi-fusionnée est de formule (I) selon l'une des revendications 1-7 ou est susceptible d'être obtenu par le procédé selon l'une des revendications 8-11, dans des dispositifs et/ou matériaux de mesure et/ou de catalyse.

13. L'utilisation selon la revendication 12, dans laquelle une production d'hydrogène photocatalytique, à partir d'une dissociation de l'eau, avec le film polymère de porphyrine multi-fusionnée est supérieure ou égale à environ 5 µmol.cm⁻².h⁻¹, même supérieure ou égale à 9 µmol.cm⁻².h⁻¹, en particulier supérieure ou égale à 20 µmol.cm⁻².h⁻¹.
